(19) Europäisches Patentamt
European Patent Office
Office européen des brevets

(11) Publication number: 0 076 369
A1

# (12) EUROPEAN PATENT APPLICATION

(21) Application number: 82106674.3

(22) Date of filing: 02.07.79

(51) Int. Cl.³: C 07 D 498/04
//(C07D498/04, 263/00, 205/00)

(30) Priority: 09.09.78 GB 3627078

(43) Date of publication of application: 13.04.83 Bulletin 83/15

(84) Designated Contracting States: AT LU

(60) Publication number of the earlier application in accordance with Art. 76 EPC: 0 009 302

(71) Applicant: BEECHAM GROUP PLC
Beecham House Great West Road
Brentford Middlesex(GB)

(72) Inventor: Stirling, Irene
38 Harrison Close
Reigate Surrey(GB)

(72) Inventor: Clarke, Brian Peter
"Coneybury" Drive Spur Forest Drive
Kingswood Surrey(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

## (54) Beta-lactam compounds.

(57) The present invention provides the compound of formula:

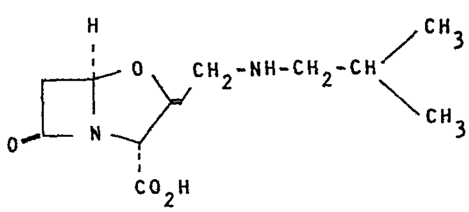

and esters thereof.

A process for the preparation of the compound comprises hydrogenation of a compound of formula (II):

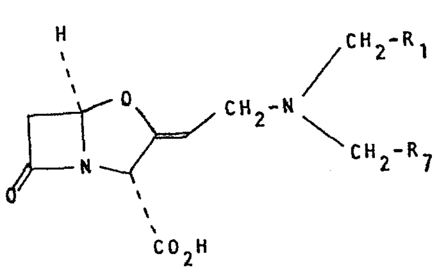

(II)

wherein $R_1$ is isopropyl or a group that on hydrogenation provides an isopropyl group, and $-CH_2R_2$ is a group removable by hydrogenation.

EP 0 076 369 A1

# β-Lactam Compounds

Our earlier cognate British Patent Application No. 16764/77 - 37072/77 - 50229/77 - 53866/77 (and also French Patent Application No. 78-11 851, West German Patent Application No. P.28 17 085.6, Japan Patent Application No. 48292/78 and U.S.A. Patent Application No. 896 441) disclosed inter alia a process for the preparation of the compounds of the formula (I):

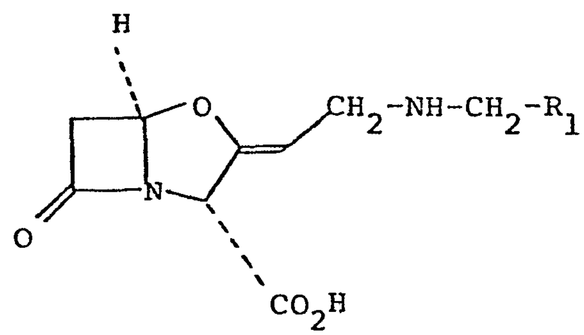

(I)

wherein $R_1$ is a hydrogen atom, an alkyl group of up to 5 carbon atoms, a cycloalkyl group of 5 or 6 carbon atoms, a hydroxyalkyl group of up to 5 carbon atoms or a moiety of the sub formula (a):

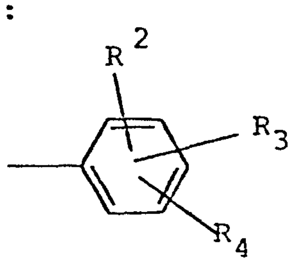

(a)

wherein $R_2$ is a hydrogen, fluorine, chlorine or bromine atom or an alkyl group of 1 - 3 carbon atoms, an alkoxyl group of 1 - 3 carbon atoms, an acyloxyl group of 1 - 3 atoms, a hydroxyl group, an alkoxycarbonyl group containing 1 - 3 carbon atoms in the alkoxy part, or a group $-N(R_5)CO.R_6$, $-N(R_5)SO_2R_6$ or $-CO-NR_5R_6$ where $R_5$ is a hydrogen atom or an alkyl groupof 1 - 3 carbon atoms or a phenyl or benzyl group and $R_6$ is an alkyl group of 1 - 3 carbon atoms or a phenyl or benzyl group; $R_3$ is a hydrogen, fluorine or chlorine atom or an alkyl group of 1 - 3 carbon atoms, an alkoxyl group of 1 - 3 carbon atoms or an acyloxyl group of 1 - 3 carbon atoms; and $R_4$ is a hydrogen fluorine or chlorine atom or an alkyl group of 1 - 3 carbon atoms or an alkoxyl group of 1 - 3 carbon atoms. The disclosed process comprised inter alia the hydrogenation of a compound of the formula (II):

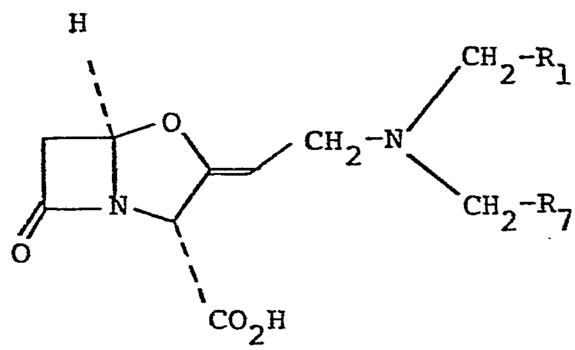

(II)

or a hydrogenolysable ester thereof wherein $R_1$ is as defined in relation to formula (I) and $R_7$ is a group of the sub-formula (a) as defined in relation to formula (I)

It has now been discovered that the compounds of the formula (I) may also be prepared by the catalytic removal of substituted allyl groups from compounds of analogous to those of formula (II) in which the $CH_2R_7$ moiety is replaced by a substituted allyl group. This process can improve yields of product and can be more convenient in that quicker reaction times and lower

pressures of hydrogen may be employed.

Thus the compounds of formula (I) may be prepared by a process which comprises the catalytic hydrogenation of a compound of the formula (III):

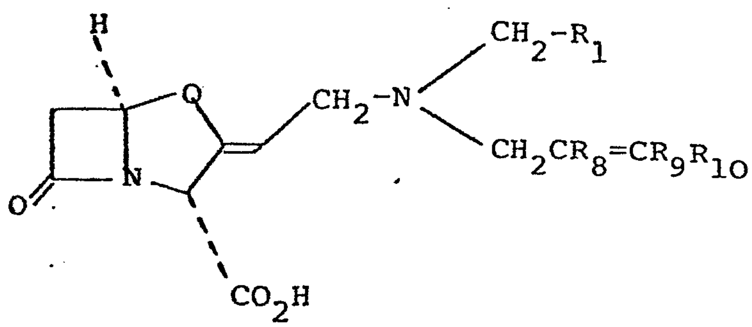

(III)

or a hydrogenolysable ester thereof wherein $R_1$ is as defined in relation to formula (I), or $R_1$ is $R_1^{\,1}$ is a moiety that on hydrogenation provides a group $R_1$ as defined in formula (I), $R_8$ is a lower alkyl group or a hydrogen atom, $R_9$ is a hydrogen atom or a lower alkyl group and $R_{10}$ is a hydrogen atom or a lower alkyl or phenyl group

When used herein the term "hydrogenolysable ester" means an ester which on hydrogenation is cleaved to yield the parent carboxylic acid.

When used herein the term "lower alkyl" means an alkyl group of up to 4 carbon atoms. Thus examples of suitable alkyl groups are the methyl, ethyl, n-propyl, iso-propyl, n-butyl, sec-butyl and iso-butyl groups.

Certain compounds of formula (I) are novel. In particular, the present invention provides the compound of formula (I) wherein $R_1$ is isobutyl, namely 9-N-isobutylaminodeoxyclavulanic acid or an ester thereof.

Particularly apt groups $CH_2CR_8=CR_9R_{10}$ for use in the compounds of the formula (III) include the following $CH_2C(CH_3)=CH_2$, $CH_2C(C_2H_5)=CH_2$, $CH_2C(n.C_3H_7)=CH_2$, $CH_2C(CH_3)=CH.CH_3$, $CH_2C(CH_3)=C(CH_3)_2$, $CH_2C(CH_3)=CH.C_2H_5$ and $CH_2.C(CH_3)=CH.C_6H_5$.

A favoured group $CH_2$ $CR_8=CR_9R_{10}$ is the $CH_2C(CH_3)=CH_2$ group.

A further favoured group $CH_2CR_8=CR_9R_{10}$ is the $CH_2C(CH_3)=CHPh$ group.

The compound of the formula (III) may be pre-formed although it is normally produced in-situ by the hydrogenation of a hydrogenolysable ester. It follows that it is a preferred form of the process to prepare the compound of this invention to employ a hydrogenolysable ester of a compound of the formula (III).

Hydrogenolysable esters for use in this invention are generally benzyl or substituted benzyl esters: and also optionally substituted allyl esters. Suitable esters include those of the part-formula (g): $CO_2CHA_1A_2$ wherein $A_1$ is a hydrogen atom or a lower alkyl or optionally substituted phenyl group and $A_2$ is an optionally subsituted phenyl group.

Favourably $A_1$ is a hydrogen atom.

The nature of the substituent employed in a substituted phenyl group is unimportant as long as it does not interfere with the hydrogenolytic cleavage. Thus

suitable substituents include lower alkyl, lower alkoxyl, lower acyloxyl, lower acyl, nitro, cyano, carboxylic acid groups or salts or lower alkyl esters or amides thereof, nitro, halo or similar substituents. Apt substituents include methyl, methoxyl, nitro, chloro, bromo and the like. One, two or three such substituents may be employed (except not more than one nitro group should be present).

Favoured esters include the benzyl, nitrobenzyl, bromobenzyl, chlorobenzyl, methylbenzyl and methoxybenzyl esters. Particularly favoured esters include the benzyl, p-nitrobenzyl and p-methoxybenzyl sters. The preferred ester is the benzyl ester.

Further preferred hydrogenolysable ester groups include those groups $CH_2CR_8 = CHR_{10}$ that have been specified hereinbefore as being favoured for removal from a nitrogen atom by hydrogenolysis.

The hydrogenation reaction is normally carried out in the presence of a transition metal catalyst.

The catalyst we have preferred to use is palladium, for example in the form of palladium on carbon (charcoal), palladium on barium sulphate, palladium on calcium carbonate, palladium black or the like.

A favoured catalyst is palladium on carbon (sometimes referred to as palladium on charcoal); for example 5%, 10%, 20% or 30% palladium on carbon.

The higher palladium content catalyst can be particularly apt as smaller total weights of catalyst can be employed thereby avoiding possible problems associated with adsorption of product onto the carbon.

Alternatively use of a catalyst containing a lower proportion of metal and a higher quantity of carbon can be used specifically to adsorb the product. This aids in isolation as the product may be recovered therefrom thereafter by washing.

A low, medium or high pressure of hydrogen may be used in this reaction, for example from 1 to 6 atmospheres.

However it is one of the considerable advantages of the process that is proceeds smoothly and quickly even at atmospheric pressure. Thus a particularly favoured aspect of the process comprises carrying out the hydrogenation at atmospheric pressure.

The reaction is normally carried out at a non-extreme temperature, for example from $0^{o}C$ to $30^{o}C$ and more usually from $12^{o}C$ to $25^{o}C$. It is generally convenient to carry out the reaction at ambient temperature.

Suitable solvents for carrying out the hydrogenation include ethanol, n-propanol, isopropanol, tetrahydrofuran, dioxan , ethyl acetate or mixtures of such solvents or such solvents in the presence of water. A favoured solvent is ethanol.

The product may generally be isolated from the reaction mixture by filtering off the solids (the catalyst, which should be well washed to remove the product) and then evaporating the solvent, preferably under low pressure, to yield the initial product. Further purification may be effected by such conventional methods as chromatography over cellulose or other mild stationary phase eluting with a $C_{1-4}$ alkanol optionally in the presence of water and optionally in the presence of tetrahydrofuran. Evaporation of the combined active fraction (identified by aqueous potassium permanganate spray on tlc) then yields the desired compound in pure form.

The desired product of the invention is normally obtained in crystalline form (unless it is an unsalted ester). Trituration under ethanol, idopropanol or the like $C_{1-4}$ alkanol or other conventional solvent such as a ketone, ether or ester solvent or other conventional solvent (for example of up to 6 carbon atoms and more suitably of up to 4 carbon atoms) may also be used to aid crystallisation. Recrystallisation from ethanol or the like may also be employed. The solvent used in such processes may advantageously be moist.

The compounds of the formula (III) and their hydrogenolysable esters may be prepared by the methods set forth in British Patent Application No. 41887/75, U.S. Serial No. 731928, Belgian Patent No. 847044 and West German Offenlegungsschrift No. 26460037. In summary an apt form of this process comprises the reaction of an hydrogenolysable ester of an acyl derivative of clavulanic acid with an amine of the formula (IV):

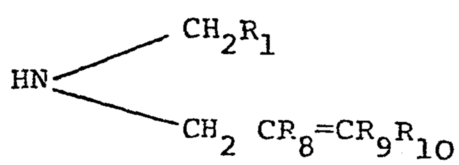

(IV)

wherein $R_1$, $R_8$, $R_9$, and $R_{10}$ are as defined in relation to formula (III) and thereafter cleaving the ester if desired.

A favoured acyl derivative is the dichloroacetate. A favoured ester is the benzyl ester.

The following Examples illustrate the invention.

Example 1

Benzyl 9,N,N-bis(2'-methylallyl)aminodeoxyclavulanate

Benzyl dichloroacetylclavulanate (5 g; 12.5 mm) in dry dimethylformamide (75 $cm^3$) at $0^o$ was treated with bis (2-methylallyl)amine (1.9 equivalents) and stirred at $0^o$ for 2 hours. The mixture was poured into ethylacetate (250 $cm^3$) and washed with water (5 x 100 $cm^3$) and saturated brine (5 x 100$cm^3$), dried (anhydrous magnesium sulphate) and evaporated to an oil. This oil was chromatographed on silica eluting with ethylacetatecyclohexane (1:1). Fractions were collected containing the title compound Rf ($SiO_2$/ethylacetate-cyclohexane; 1:1) = 0.82 (detection by aqueous potassium permanganate spray). Combined fractions were evaporated to yield an oil, 1.46 g (29%).

υ (film) 1805, 1750, 1695, 895, 755, 700 $cm^{-1}$. δ ($CDCl_3$) 1.67 (6H, s), 2.78 (4H, s), 2.95 (1H, d, J 17Hz), 3.03 (2H, d, J 7Hz), 3.42 (1H, dd, J 17 and 3Hz), 4.67 (1H, t, J 7Hz), 4.80 (4H, broads), 5.03 (1H, s), 5.17 (2H, s), 5.61 (1H, d, J 3Hz), 7.33 (5H, s).

Example 2

9-N-Isobutylaminodeoxyclavulanic acid

Benzyl 9-N,N-bis(2'-methylallyl)aminodeoxyclavulanate (0.8 g; 2.02 mm) in ethanol (30 $cm^3$) was hydrogenated in the presence of palladium on charcoal 10% (0.3 g) for 10 min at $1.013x10^5$ Pa (1 atmosphere) the catalyst had been pre-hydrogenated for 20 minutes. The catalyst was filtered off and washed with aqueous ethanol (50 $cm^3$), the filtrate was evaporated in vacuo and ethanol added, after cooling at $0^o$ a crystalline solid was filtered off and washed with cold ethanol, drying in vacuo afforded the title compound as a white crystalline solid; yield = 190 mg (37%) Rf ($SiO_2$/butanol-propan-2-ol-water; 7:7:6) = 0.45 $\nu$ (Nujol) 1805, 1690, 1610, 1570, 1185, 1110, 1080, 1070, 1045, 1020, 1005, 930, 895, 885, 857, 810, 758 $cm^{-1}$. $\nu$ (KBr) 3570 (broad), 3350 (broad), 3220 (broad), 2840-2740, 2450 (broad), 1780, 1695, 1600 (very broad), 1470, 1392, 1320, 1295, 1110, 1045, 1020, 1003, 987, 932, 893, 885, 812, 750 $cm^{-1}$. $\delta$ ($D_2O$) 0.94 (6H, d, J, 6Hz), 1.90 (1H, m), 2.82 (2H, d, J 6Hz), 3.07 (1H, d, J 17Hz), 3.57 (1H, dd, J 17 and 3 Hz), 3.70 (2H, d, J 17 and 3 Hz), 3.70 (2H, d, J 7Hz), 4.77 (1H, t, J 7Hz), 4.96 (1H, d), 5.73 (1H, d, J 3Hz).

Example 3

Benzyl 9-N-isobutyl-N-(2'-methyl-3'-phenylallyl)aminodeoxyclavulanate

Benzyl dichloroacetylclavulanate (7.16 g; 17.9 mM) in dry dimethylformamide (85 $cm^3$) at $-12^o$ was treated with 1.9 equivalents of N-isobutyl-N-(2-methyl-3-phenylallyl) amine and stirred at $-15^o$ for 15 minutes then for 45 minutes between $-10^o$ and $0^o$. The mixture was poured into iced ethylacetate (250 $cm^3$) and washed with water (5 x 100 $cm^3$) saturated brine (5 x 100 $cm^3$), dried (anhydrous magnesium sulphate) and evaporated in the presence of toluene to a small volume. This crude product was chromatographed on silica eluting with ethylacetate - cyclohexane; 1:2. Fractions were collected containing the title compound Rf ($SiO_2$/ethylacetate - cyclohexane; 1:2) = 0.89. Combined fractions were evaporated to afford the title compound as as oil, yield = 1.60 g (19%), $\nu$ (film) 1808, 1750, 1690, 745, 700 $cm^{-1}$. $\delta$ ($CDCl_3$) 1.85 (6H, d, J 6 Hz), 1.50 - 2.20 (6H, broad m), 2.92 (1H, d, J 17 Hz), 2.94 (2H, s), 3.39 (1H, dd, J 17 and 3 Hz), 4.73 (1H, broad, t, J 7 Hz), 5.07 (1H, broad s), 5.17 (2H, s), 5.60 (1H, d, J 3 Hz), 6.35 (1H, broad s), 7.25 and 7.32 (10 H, 2 x s).

Example 4

9-N-Isobutylaminodeoxyclavulanic acid

Benzyl 9-N-(2'-methyl-3'-phenylallyl)-N-isobutylaminodeoxyclavulanate (1.44 g; 3.04 mM) in ethanol (25 $cm^3$) was hydrogenolysed in the presence of palladium on carbon (10% Pd), 0.5 g (which had been prehydrogenated for 15 minutes), for 45 minutes at $1.013x10^5$Pa (1 atmosphere). The catalyst was filtered off and washed with ethanol (50 $cm^3$), then with aqueous ethanol (100 $cm^3$), the aqueous washing was collected separately and was evaporated to yield the title compound as a white crystalline solid. The solid was washed with a little cold ethanol, drying afforded 248 mg of the title compound. The ethanolic catalyst washing and the solvent from the hydrogenolysis were evaporated, ethanol added (10 $cm^3$) and cooled, crystals were filtered off and dried to yield a further 17 mg of the title compound, total yield = 265 mg (34%). The infrared and proton magnetic resonance spectra were identical to the product obtained by hydrogenolysis of benzyl 9-N-N-bis(2'-methylallyl)aminodeoxyclavulanate.

Example 5

Benzyl 9-N-(2'-methylallyl)-N-(3"-methylallyl)aminodeoxyclavulanate

Benzyl dichloroacetylclavulanate (7.4 g; 18.5 mM) in dry dimethylformamide (50 $cm^3$) at $0^o$ was treated with N-(2'-methylallyl)-N-(3"-methylallyl) amine (1.9 equivalents) and stirred at $0^o$ for 45 minutes. The mixture was poured into ethyl acetate (200 $cm^3$) and washed with water (6 x 100 $cm^3$) and saturated brine (6 x 100 $cm^3$), dried (anhydrous magnesium sulphate) and evaporated to an oil. This oil was chromatographed on silica eluting with ethyl acetate - cyclohexane (1:2), fractions were collected Rf ($SiO_2$/ethyl acetate - cyclohexane; 1:2) = 0.5 (detection by aqueous potassium permanganate spray). Combined fractions were evaporated to yield the title compound as an oil, yield = 2.7 g (37%). υ (film) 1805, 1750, 1700, 1450, 1380 1305, 1230, 1175, 1120, 1080, 1042, 1015, 970,895, 740, 700 $cm^{-1}$. δ ($CDCl_3$) 1.58-1.80 (6H, m), 2.81 (4H, broads), 2.95 (1H, d, J 17 Hz), 3.08 (2H, d, J 7 Hz), 3.42 (1H, dd, J 17 and 3 Hz), 4.68 (1H, t, J 7 Hz), 4.79 (2H, broad s), 5.03 (1H, s), 5.15 (2H, s), 5.36-5.56 (2H, broad m), 5.60 (1H, d, J 3 Hz), 7.32 (5H, s).

Example 6

9-N-Isobutylaminodeoxyclavulanic acid

Benzyl 9-N-(2'-methylallyl)-N-(3'-methylallyl)aminodeoxyclavulanate (137mg) in ethanol (30 $cm^3$) was hydrogenolysed at $1.013x10^5$Pa (1 atmosphere) in the presence of 10% palladium on charcoal (0.5 g which had been prehydrogenated for 20 minutes) for 25 minutes. The catalyst was filtered off and washed with aqueous ethanol, the filtrate was evaporated and ethanol added. The resulting crystals were filtered off cold and dried. This product was chromatographed on cellulose eluting with butanol-isopropanol-water; 4:4:1. Fractions were collected containing the title compound in low yield Rf($SiO_2$/butanol-isopropanol water; 7:7:6)= 0.46 (detection by aqueous potassium permanganate spray).

Example 7

Benzyl 9-N-(2'-methylallyl)-N-(3"-transphenylallyl) aminodeoxyclavulanate

Benzyl dichloroacetylclavulanate (5.9g; 14.8 mM) in dimethylformamide (40 $cm^3$) at $0^o$ was treated with N-(2'-methylallyl)-N-(3'-transphenylallyl) amine (1.9 equivalents) and stirred at $0^o$ for $1^3/_4$ hours. The mixture was then poured into ethyl acetate (250 $cm^3$) and washed with water (5 x 100 $cm^3$) and saturated brine (5 x 100 $cm^3$), dried (anhydrous magnesium sulphate) and evaporated to an oil. This oil was chromatographed on silica eluting with ethylacetate-cyclohexane (1:2), fractions were collected containing the title compound; Rf($SiO_2$/ethylacetate:cyclohexane 1:1) = 0.76 and combined fractions were evaporated to yield an oil, 3.49 g (51%). Detection by aqueous potassium permanganate spray. $\nu$ (film) 1805, 1750, 1700, 1495, 1450, 1305, 1230, 1175, 1120, 1080, 1045, 1015, 967, 895, 745, 695 $cm^{-1}$. $\delta$ ($CDCl_3$) 1.71 (3H, s), 2.89 (2H, s), 2.93 (1H, d, J 17Hz), 3.07 (2H, d, J 6Hz), 3.16 (2H, d, J 7Hz), 3.39 (1H, dd, J 17 and 3Hz), 4.72 (1H, t, J 7Hz), 4.75-4.95 (2H, broad m), 5.06 (1H, d, J 3Hz), 5.16 (2H, s), 6.15 (1H, dt, J 16 and 6Hz), 6.45 (1H, d, J 16Hz), 7.15-7.50 (10H, m).

Example 8

9-N-Isobutylaminodeoxyclavulanic acid

Benzyl 9-N-(2'-methylallyl)-N-(3"-trans phenylallyl) aminodeoxyclavulanate (11g) in ethanol (40 $cm^3$) was hydrogenolysed for 20 minutes at $1.013 \times 10^5$Pa (1 atmosphere) in the presence of 10% palladium on charcoal (300 mg; which had been prehydrogenated for 10 minutes). The catalyst was filtered off and washed with aqueous ethanol. The filtrate was evaporated and ethanol added, the resulting crystals were filtered off cold and dried. This product was chromatographed on cellulose eluting with butanol-isopropanol water; 4:4:1. Fractions were collected containing the title compound in low yield Rf($SiO_2$/butanol-isopropanol-water; 7:7:6) = 0.46 (detection by aqueous potassium permanganate spray).

Example 9

Benzyl 9-N-(2'-methylallyl)-N-(2"-methyl-3"-phenylallyl) aminodeoxyclavulanate

Benzyl 9-O-dichloroacetylclavulanate (6.30 g; 15.8 mmole) in acetonitrile (60 ml) at $4^oC$ was treated with dropwise addition of N-(2'-methylallyl)-N-(2"-methyl-3"-phenylally) amine (6 g; 30 mmol) in acetonitrile (60 ml). On final addition the reaction was stirred at 4 to $10^oC$ for 2 hours.

The acetonitrile was then removed under a reduced pressure and the resulting oil was dissolved in ethyl acetate. The solution was then washed with water, brine, dried ($MgSO_4$) and evaporated *in vacuo*. Silica-gel column chromatography afforded the title compound as as an oil in a 16% yield.

$\nu_{max}$ ($CHCl_3$): 1802, 1745, 1695 and 1600 (br) $cm^{-1}$.

$\delta$ ($CDCl_3$): 1.70 (3H, s), 1.82 (3H, s), 2.84 (2H, s), 2.92 (2H, s), 2.90(1H, d, *J* 17 Hz), 3.12 (2H, d, *J* 7 Hz), 3.38 (1H, dd, *J* 17 and 3 Hz), 4.70 (1H, br.t, *J* 7 Hz), 4.82 (2H, br.s), 5.06 (1H, s), 5.16 (2H, s), 5.60 (1H, d, *J* 3 Hz), 6.36 (1H, s), 7.22 and 7.30 (10H, 2 x s).

Example 10

9-N-Isobutylaminodeoxyclavulanic acid

Benzyl-9-N-(2'-methylallyl)-N-(2"-methyl-3"phenylallyl) aminodeoxyclavulanate (1.0 g; 2 mmol) in ethanol (20 ml) was carefully added to a pre-hydrogenated mixture of 10% palladium on charcoal (300 mg) in ethanol (30 ml). The mixture was then hydrogenated at $1.013x10^5$Pa (1 atmosphere) for 30 minutes. The catalyst was then filtered off and washed well with aqueous ethanol. The filtrate plus washings were then evaporated to dryness under a reduced pressure. Cellulose column chromatography afforded the title compound as a white crystalline solid in 30% yield.

Example 11

Benzyl 9-N-(iso-butyl)-N-(2'-methylallyl)aminodeoxyclavulanate

N-Isobutyl-N-(2-methylallyl)amine (7.25 g, 57 mmol) in dimethylformamide (30 ml) was added dropwise to a solution of benzyl dichloroacetylclavulanate (12.0 g, 30 mmol) in dimethylformamide (200 ml) at -10°C. After 2 hours at this temperature the reaction mixture was poured into water and extracted with ethylacetate. The organic phase was washed several times with brine, dried ($MgSO_4$) and evaporated to a yellow oil. Chromatography in silica gel (elution : petrol/ethylacetate : 3/1 grading to 2/1) afforded the title ester as an oil, 2.98 g (25%)

I.R. ($CHCl_3$) 1802, 1745, 1700, and 895 $cm^{-1}$

N.M.R. ($CDCl_3$) 0.84 (6H, d, J 7 Hz), 1.52-1.88 (1H, m) 1.68 (3H, s), 2.00 (2H, d, J 7 Hz), 2.79 (2H, s), 2.94 (1H, d, J 17 Hz), 3.06 (2H, d, J 8 Hz), 3.41 (1H, dd, J 17 and 3 Hz), 4.68 (1H, bt, J 8 Hz), 4.78 (2H, bs), 5.03 (1H, bs), 5.17 (2H, s), 5.61 (1H, d, J 3 Hz), and 7.32 (5H, s).

Example 12

9-N-Isobutylaminodeoxyclavulanic acid

10% Pd-C (0.83 g) in ethanol (80 ml) was hydrogenated for 15 minutes at $1.013 \times 10^5$ Pa (1 atmosphere). Benzyl 9-N-(isobutyl)-N-(2'-methylallyl)aminodeoxyclavulanate (2.5 g, 6.28 mmol) in ethanol (30 ml) was added and the hydrogenation continued for 45 minutes. The catalyst was then filtered off through celite and the pad was washed with some ethanol. These combined washings were evaporated to a yellow oil which crystallised from ethanol (0.120 g).

The filter pad, above was now washed with 50% aqueous ethanol (150 ml). Evaporation afforded the title material as a white solid (0.70 g).

Combination of all the mother liquors and evaporation afforded a dark oil which was chromatographed on silica gel (elution : ethylacetate/isopropanol/water : 5/2/1 grading to 5/4/3) to provide more of the title product (0.115 g).

Total yield of required product = 0.935 g (59% yield).

N.M.R., I.R , and t.l.c. characteristics were identical to an authentic sample.

Example 13

Phenacyl 9-N-Bis (2'-methylallyl)aminodeoxyclavulanate

Phenacyl 9-O-dichloroacetylclavulanate (5.0 g; 11.7 mmol) in dry dimethylformamide (50 ml). The mixture was cooled to $0^oC$ and bis (2'-methylallyl) amine (2.8 g; 22.2 mmol) in dry dimethylformamide (30 ml) was added dropwise. After stirring at $0^oC$ for 1½ hours the mixture was poured into ethyl acetate and washed several times with water and then dried ($MgSO_4$). After filtration the ethyl acetate was removed *in vacuo* to give a yellow oil, which on column chromatography afforded the required compound as a yellow gum, yield 32%.

$[\alpha]_D^{20}$+ 11.0° (c. 1%, $CHCl_3$). Found C, 67.60; H, 6.70; N 6.38%, $C_{24}H_{28}H_2O_5$ requires: C, 67.91; H, 6.65; N, 6.60% $\nu_{max}$ ($CHCl_3$) 1805, 1760, and 1605 $cm^{-1}$; $\delta$ ($CDCl_3$) 1.72 (6H, s), 2.76→3.22 (7H, m), 3.45 (1H, dd, J 17 and 3 Hz), 4.82 (5H, m), 5.19 (1H, s), 5.38 (2H, s), 5.66 (1H, d, J 3 Hz), 7.30→7.60 and 7.78→8.00.

Example 14

Allyl 9-N,N-bis(2'-methylallyl)aminodeoxyclavulanate

Allyl dichloroacetylclavulanate (5 g; 14.3 mM) in dry dimethylformamide (50 $cm^3$) at $0^o$ was treated with bis (2 methylallyl)amine (1.9 equivalents) and stirred 2½ hours at between $0^o$ and $5^oC$. The mixture was poured into ethylacetate (250 $cm^3$) and washed with water (5 x 200 $cm^3$) and saturated brine (5 x 150 $cm^3$), dried (anhydrous magnesium sulphate) and evaporated to an oil. This oil was chromatographed on silica eluting with ethylacetate - cyclohexane; 1:1. Fractions were collected containing the title compound, Rf ($SiO_2$/ethylacetate - cyclohexane; 1:1) = 0.90 (detection by aqueous potassium permanganate spray). Combined fractions were evaporated *in vacuo* to yield an oil, 1.33 g (27%), (film) 1808, 1750, 1695, 1450, 1370, 1308, 1232, 1180, 1120, 1015, 940, 895 $cm^{-1}$.

($CDCl_3$) 1.71 (6H, s), 2.83 (4H, s), 2.98 (1H, d, *J* 17 Hz), 3.07 (2H, d, *J* 7 Hz), 3.45 (1H, dd, *J* 17 and 3 Hz), 4.55-4.94 (7H, m), 5.02 (1H, s), 5.17 - 5.5 (2H, m), 5.63 (1H, d, *J* 3 Hz), 5.6 - 6.16 (1H, m).

Example 15

(2-Methylallyl) 9'-N-(Isobutyl)-N-(2"-Methylallyl) aminodeoxyclavulanate

(2-Methylallyl)-9'-O-dichloroacetylclavulanate (9.05 g; 24.8 mmol) in anhydrous dimethylformamide (90 ml) was cooled with stirring to -20°C. N-(Isobutyl)-N-(2-methylallyl) amine (6.0 g; 47 mmol) in anhydrous dimethylformamide (60 ml) was added dropwise over a period of 20 minutes. The reaction mixture was allowed to warm to -10°C and stirring continued for 2 hours.

The solution was poured into a cold mixture of ethyl acetate and water and shaken. The aqueous layer was extracted with more ethyl acetate. The combined organic layers were washed with water, dried ($MgSO_4$) and evaporated to an oil. Column chromatography afforded the title compound as a colourless oil in 23% yield.

$\nu_{max}$ ($CHCl_3$): 1805, 1750 and 1700 $cm^{-1}$. $\delta$($CDCl_3$): 0.85 (6H, d, *J* 7Hz), 1.70 (3H, s), 1.77 (3H, s), 1.57 to 1.88 (1H, m), 2.05 (2H, d, *J* 7Hz), 2.84 (2H, s), 2.94 (1H, d, *J* 17Hz), 3.11 (2H, d, *J* 7Hz), 3.46 (1H, dd, *J* 17 and 3Hz), 4.58 (2H, s), 4.74 (1H, broad t, *J* 7Hz), 4.81 (2H, broad s), 4.99 (2H, broad s), 5.06 (1H, s), and 5.66 (1H, d, *J* 3Hz).

Example 16

9-N-Isobutylaminodeoxyclavulanate

(2-Methylallyl)-9'-N-(isobutyl)-N-(2"-methylallyl) aminodeoxyclavulanate (1.5g; 4 mmol) in ethanol (20ml) was carefully added to a pre-hydrogenated mixture of 10% palladium on charcoal (0.5g) in ethanol (20 ml). The mixture was hydrogenated at $1.013x10^3$Pa (1 atmosphere) until the uptake of hydrogen ceased.

The mixture was filtered through a celite pad and the "cake" washed with aqueous ethanol. The filtrate plus washings were evaporated to dryness resulting in a yellow solid which an addition of a small amount of ethanol gave a white solid. Filtration afforded the title compound in 20% yield. Spectral data was consistent with an authentic example.

CLAIMS (for Luxembourg)

1. The compound of formula:

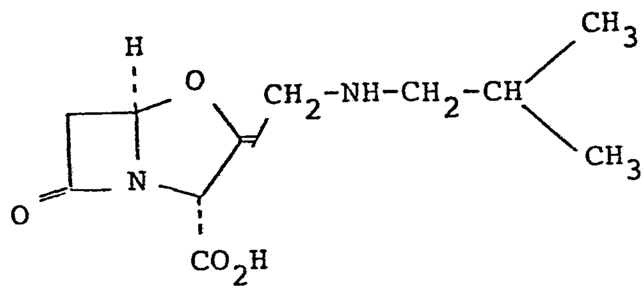

or an ester thereof.

2. 9-N-Isobutylaminodeoxyclavulanic acid.

3. 9-N-Isobutylaminodeoxyclavulanic acid when in crystalline form.

CLAIMS (for Austria)

1. A process for the preparation of a compound of formula:

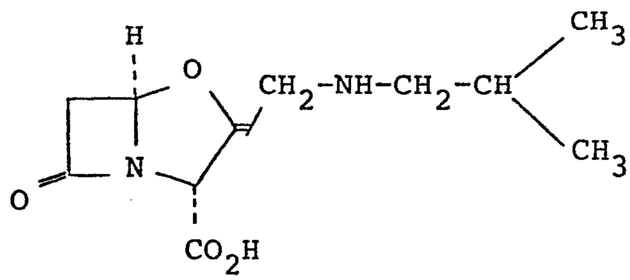

or an ester thereof which process comprises the hydrogenation of a compound of formula:

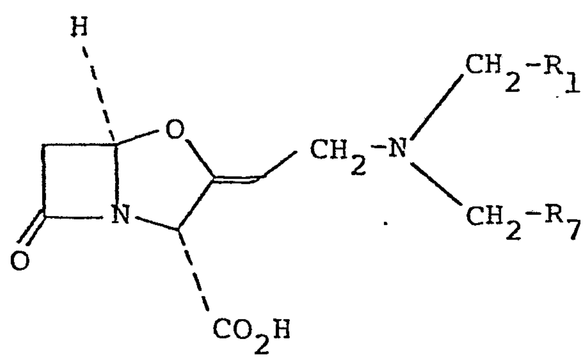

or a hydrogenolysable ester thereof, wherein $R_1$ is isopropyl or a group that on hydrogenation provides an isopropyl group, and $-CH_2R_1$ represents a group removable by hydrogenation.

(Austria)

2. A process as claimed in claim 1 wherein $R_1$ represents isopropyl or isopropylene.

European Patent Office

# EUROPEAN SEARCH REPORT

0076369

Application number

EP 82 10 6674

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| | --- | | C 07 D 498/04 // (C 07 D 498/04 C 07 D 263/00 C 07 D 205/00 ) |
| Y | DE-A-2 646 003 (BEECHAM) *Claim 1* | 1 | |
| | --- | | |
| D,P Y | DE-A-2 817 085 (BEECHAM) *Claims 1,3,6,36,37* | 1 | |
| | ----- | | |
| | | | **TECHNICAL FIELDS SEARCHED (Int. Cl. 3)** |
| | | | C 07 D 498/00 A 61 K 31/00 |

The present search report has been drawn up for all claims

| Place of search | Date of completion of the search | Examiner |
|---|---|---|
| THE HAGUE | 02-12-1982 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document

EPO Form 1503. 03.82